# EUROPÄISCHE PATENTANMELDUNG

(11) **EP 1 226 780 A2**
(43) Veröffentlichungstag der Anmeldung: **31.07.2002**
(21) Anmeldenummer: 02000802.5
(22) Anmeldetag: 14.01.2002
(51) Int. Cl.: A61B 5/00, A61B 5/16

(54) **Verfahren, Vorrichtung und tragbares Gerät zur Bestimmung psychomotorischer Fähigkeiten**

(30) Priorität: 25.01.2001 DE 10103326
(71) Anmelder: SIEMENS AKTIENGESELLSCHAFT, 80333 München (DE)
(72) Erfinder: Schmidt, Volker, Dr., 91054 Erlangen (DE); Striebel, Werner, 90592 Schwarzenbruck (DE)

(57) **Zusammenfassung**

Die Erfindung betrifft ein Verfahren und Vorrichtungen zur Bestimmung psychomotorischer Fähigkeiten eines Patienten (1) in außerklinischer Umgebung (5), aufweisend eine Datenbank (3), welche an einem Ort (6) angeordnet ist, der von dem Ort (5), an dem ein psychomotorischer Test zur Bestimmung der psychomotorischen Fähigkeiten durchgeführt wird, verschieden ist, und welche während des psychomotorischen Tests ermittelte Daten des Patienten (1) speichert. Die Erfindung betrifft außerdem ein tragbares Gerät zur Bestimmung psychomotorischer Fähigkeiten eines Patienten, aufweisend eine Schreibfläche (21), auf der handschriftlich Buchstaben oder Zahlen geschrieben werden können, Mittel (22) zum Erkennen der handschriftlich geschriebenen Buchstaben oder Zahlen, ein Sichtfeld (23) zum Wiedergeben der erkannten handschriftlichen Buchstaben oder Zahlen als Druckbuchstaben und Mittel (24) zum Anschluss an das Internet.

## Beschreibung

Die Erfindung betrifft ein Verfahren, Vorrichtungen und ein tragbares Gerät zur Bestimmung psychomotorischer Fähigkeiten eines Patienten.

Bei einer medikamentösen Behandlung eines Patienten mit an seinem Nervensystem angreifenden Substanzen ist es nötig, psychische und motorische Fähigkeiten des Patienten während der Behandlung zu überprüfen, um insbesondere Unter- oder Überdosierungen rechtzeitig zu erkennen und Nebenwirkungen der Substanzen zu minimieren. Am Zentralnervensystem angreifende Substanzen umfassen u.a. Sedativa (Schlaftabletten), Amphetamine (Wachmacher, Appetitzügler), Neuroleptika, Antidepressiva und Antiepileptika. Die Wirkungen dieser Substanzen können sich u.a. in einer Veränderung der Vigilanz, der Reaktionszeit, der Fein- und Grobmotorik und des Erinnerungsvermögens des Patienten bemerkbar machen.

Um eine angemessene Behandlung des Patienten zu gewährleisten, kann beispielsweise die Konzentration der eingenommenen Substanzen im Blut des Patienten labortechnisch bestimmt werden. Zusätzlich werden in einem Krankenhaus oder in einer Arztpraxis spezielle Tests beispielsweise zur Bestimmung der Reaktionszeit, der psychomotorischen Koordinationsfähigkeit und der zeitlichen und örtlichen Orientierungsfähigkeit des Patienten durchgeführt. Solche Tests können beispielsweise mit einer aus der US 6,261,239 B1 bekannten Vorrichtung zur Erfassung der koordinativen Fähigkeiten einer Testperson durchgeführt werden. Mit einer Steuerung der Vorrichtung führt die Testperson unterschiedliche koordinative Tests durch. Die Steuerung wertet die durch die Tests gewonnenen Testergebnisse aus und fasst sie zu einer quantitativen Gesamtbewertung der koordinativen Fähigkeiten der Testperson zusammen. Die quantitative Gesamtbewertung wird danach auf einem Bildschirm der Vorrichtung angezeigt.

Insbesondere bei wiederholten Tests muss der Patient öfter das Krankenhaus oder die Arztpraxis aufsuchen, was mit einem erhöhten Zeitaufwand für den Patienten verbunden ist. Deshalb sind manche Patienten geneigt, nur wenige dieser Tests durchzuführen oder Arzttermine ausfallen zu lassen, was wiederum deren Behandlungserfolge beeinträchtigt.

Der Erfindung liegt daher die Aufgabe zugrunde, ein Verfahren anzugeben sowie eine Vorrichtung derart auszubilden, mit dessen bzw. deren Hilfe Voraussetzungen geschaffen werden, Tests zur Bestimmung psychomotorischer Fähigkeiten des Patienten für den Patienten praktischer durchzuführen.

Nach der Erfindung wird diese Aufgabe gelöst durch ein Verfahren zur Bestimmung psychomotorischer Fähigkeiten eines Patienten in außerklinischer Umgebung, aufweisend folgende Verfahrensschritte:
- Kontaktieren einer Datenbank mit einem Telefon, mit einem an ein Informationsübertragungsnetz anschließbaren Rechner oder mit einem Personal Digital Assistant (PDA),
- Durchführen eines psychomotorischen Tests mit dem Telefon, dem Rechner oder dem Personal Digital Assistant (PDA), und
- Übermitteln der während des psychomotorischen Tests gewonnen Daten an eine Datenbank, welche an einem Ort angeordnet ist, der von dem Ort, an dem der psychomotorische Test durchgeführt wird, verschieden ist

Erfindungsgemäß wird also ein Test zur Bestimmung von psychomotorischen Fähigkeiten des Patienten in außerklinischer Umgebung durchgeführt. Unter außerklinischer Umgebung wird verstanden, dass der psychomotorische Test nicht in einer Arztpraxis oder in einem Krankenhaus, sondern beispielsweise beim Patienten daheim, am Arbeitsplatz des Patienten oder auch im Freien durchgeführt wird. Somit entfällt für den Patienten ein zeitaufwendiges Aufsuchen der Arztpraxis oder des Krankenhauses. Die während des Tests gewonnenen Daten werden anschließend an die zentrale Datenbank übermittelt, welche beispielsweise in der Arztpraxis eines den Patienten behandelnden Arztes angeordnet ist. Aufgrund der zentralen Speicherung der Daten ist es insbesondere möglich, dass mehrere weitere Personen, insbesondere weitere den Patienten behandelnde Ärzte, Zugriff auf die Daten haben. Der psychomotorische Test wird mit dem Telefon, dem an das Informationsübertragungsnetz anschließbaren Rechner oder mit dem Personal Digital Assistant (PDA) durchgeführt. Unter einem Personal Digital Assistant wird ein tragbares elektronisches Gerät verstanden, in das Daten eingegeben werden können, wobei der Personal Digital Assistant Mittel zur Weiterverarbeitung und Speicherung dieser Daten aufweist. Beispiele für einen Personal Digital Assistant sind ein Palmtop oder ein tragbarer Rechner (Laptop). Der Patient kann beispielsweise von einer in dem Call-Center arbeitenden Person oder automatisch von der Datenbank, welche mit geeigneten Mitteln zur Spracheingabe und Sprachwiedergabe versehen ist, telefonisch kontaktiert werden oder der Patient kann selber telefonisch eine Person des Call-Centers oder die Datenbank anrufen, damit er fernmündlich den psychomotorischen Test durchführt. Insbesondere bei der Verwendung eines Telefons kann der Test an praktisch beliebigen Orten, wie z.B. in der Wohnung des Patienten, in einer Telefonzelle oder auch im Freien, wenn der Patient insbesondere über ein mobiles Telefon verfügt, durchgeführt werden. Somit ist der Patient für die Durchführung des Tests praktisch räumlich ungebunden. Daher ergibt sich für ihn nur ein unwesentlicher, durch die eigentliche Durchführung des Tests bedingter Zeitaufwand, wodurch der Patient eher geneigt ist, diesen Test insbesondere regelmäßig oder allgemein öfter durchzuführen. Folglich wird dadurch eine Vorraussetzung für eine bessere Behandlung des Patienten geschaffen.

Gemäß einer Ausführungsform wird die Datenbank von einem Service-Anbieter betrieben. Ein solcher Service-Anbieter kann in besonders günstiger Weise die Datenbank pflegen oder insbesondere weitere Dienste anbieten. So ist es beispielsweise denkbar, dass der Service-Anbieter ein Call-Center betreibt, an das Fragen des Patienten oder des Arztes gerichtet werden können. Folglich kann eine Betreuung des Patienten effektiver gestaltet werden und insbesondere bei Fragen schnell, freundlich und zuverlässig weitergeholfen werden.

Gemäß einer weiteren Variante der Erfindung umfasst der psychomotorische Test einen Reaktionstest, einen Gedächtnistest, einen Test zur Bestimmung der Lesbarkeit der Handschrift des Patienten und/oder einen Test zur Bestimmung der Sprachverständlichkeit des Patienten. Um beispielsweise die Reaktionszeit des Patienten zu testen, können ihm Zahlen durch das Telefon genannt werden, worauf der Patient so schnell als möglich die der Zahl entsprechenden Taste bzw. Tasten an dem Telefon drückt. Das Gedächtnis des Patienten kann ähnlich getestet werden, indem dem Patienten z.B. eine Zahlenfolge genannt wird und der Patient danach die entsprechenden Tasten am Telefon drückt. Die Sprachverständlichkeit des Patienten kann beispielsweise dadurch getestet werden, dass der Patient einen dem Patienten mit dem Telefon mitgeteilten Satz oder Wortfolgen nachspricht. Ähnliche Tests können auch mit einem an das Internet anschließbaren Rechner durchgeführt werden. Mittels des Personal Digital Assistant können beispielsweise Daten, die während des psychomotorischen Tests gewonnen wurden, von dem Patienten oder einer den Patienten betreuenden Person festgehalten und später beispielsweise über das Internet der Datenbank übermittelt werden. Mit dem Personal Digital Assistant kann auch ein Test zur Überprüfung der Lesbarkeit der Handschrift des Patienten durchgeführt werden, wenn der Personal Digital Assistant eine Schreibfläche aufweist, auf der der Patient beispielsweise mit einem Stift handschriftlich einzelne Buchstaben schreibt, und der Personal Digital Assistant derart ausgeführt ist, dass er die handschriftlich geschriebenen Buchstaben erkennt und als Druckbuchstaben auf einem Sichtfeld wiedergibt. Bei einer gestörten Psychomotorik kann es sein, dass die Handschrift des Patienten unleserlich ist und der Personal Digital Assistant nur wenige handschriftlich geschriebene Buchstaben erkennt. Folglich ist eine Fehlerquote, die sich aus der Anzahl nicht erkannter Buchstaben im Vergleich zur eingegebenen Anzahl von Buchstaben errechnen lässt, ein Maß für die psychomotorische Fähigkeit des Patienten.

Gemäß einer Variante der Erfindung werden die Daten insbesondere automatisch mit einer der Datenbank zugeordneten Auswerteeinrichtung ausgewertet. Die Auswerteeinrichtung kann dabei gemäß einer vorteilhaften Variante der Erfindung Mittel zur Sprachmusteranalyse, Mittel zur Zeitmessung, Mittel zum Vergleichen von Buchstabenfolgen, Mittel zum Vergleichen von Zahlenfolgen und/oder ein Expertensystem umfassen. Mit den Mitteln zur Sprachmusteranalyse kann insbesondere der Test zur Sprachverständlichkeit des Patienten durchgeführt werden, indem der Patient wenigstens einen Satz oder eine Wortfolge, die ihm die Datenbank vorspricht, nachspricht und die Sprachverständlichkeit des nachgesprochenen Satzes bzw. der nachgesprochenen Sätze oder Wortfolge automatisch ausgewertet wird bzw. werden. Mit den Mitteln zur Zeitmessung, die beispielsweise eine Stoppuhr umfassen können, können die Reaktionszeit des Patienten bestimmt werden. Die Buchstabenfolgen bzw. Wortfolgen können mit den Mitteln zum Vergleichen von Buchstabenfolgen bzw. Zahlenfolgen ausgewertet werden.

Nach einer bevorzugten Ausführungsform ist es möglich, die Datenbank über das Internet zu kontaktieren. Somit ist es z.B. möglich, die Datenbank und somit die in der Datenbank gespeicherten Daten über das Internet abzufragen, wodurch insbesondere die den Patienten behandelnden Ärzte schnell und in einfacher Weise Zugriff auf wichtige Daten des Patienten haben.

Gemäß einer weiteren Variante der Erfindung wird der Patient und/oder wenigstens eine weitere Person insbesondere automatisch verständigt, wenn die ausgewerteten Daten bedenklich sind. Die weitere Person kann z.B. der den Patienten behandelnde Arzt sein, der aufgrund der Meldung die Daten überprüfen und wenn nötig schnell und zuverlässig angemessene Maßnahmen zur Behandlung des Patienten einleiten kann.

Eine weitere Ausführungsform der Erfindung sieht vor, dass die Datenbank automatisch den Patienten und/oder wenigsten eine weitere Person verständigt, wenn nach einem psychomotorischen Test eine vorbestimmte Zeitdauer verstrichen ist, ohne dass ein weiterer psychomotorischer Test durchgeführt wurde. Somit ist eine Vorraussetzung geschaffen, dass der Patient an die Wichtigkeit des psychomotorischen Tests erinnert werden kann, insbesondere wenn er die Durchführung des psychomotorischen Tests versehentlich vergaß.

Die Aufgabe wird auch gelöst durch eine Vorrichtung zur Bestimmung psychomotorischer Fähigkeiten eines Patienten in außerklinischer Umgebung, aufweisend
- eine Datenbank, welche an einem Ort angeordnet ist, der von dem Ort, an dem ein psychomotorischer Test zur Bestimmung der psychomotorischen Fähigkeiten durchgeführt wird, verschieden ist, und welche während des psychomotorischen Tests ermittelten Daten des Patienten speichert,
- eine der Datenbank zugeordnete Auswerteeinrichtung zur Auswertung dieser Daten und
- eine Alarmeinrichtung zur Erzeugung eines Alarmsignal, wenn die ausgewerteten Daten bedenklich sind.

Die Vorrichtung ist also dadurch gekennzeichnet, dass ein psychomotorischer Test anstelle in einer Arztpraxis oder in einem Krankenhaus insbesondere in häuslicher Umgebung, am Arbeitsplatz oder auch im Freien durchgeführt werden kann. Anschließend werden die während des psychomotorischen Tests gewonnen Daten an die zentrale Datenbank übermittelt. Die Datenbank kann beispielsweise in der Arztpraxis des den Patienten behandelnden Arzt oder einem Krankenhaus angeordnet sein. Somit braucht einerseits der Patient keine Arztpraxis oder kein Krankenhaus aufzusuchen, um den psychomotorischen Test durchzuführen, sondern kann diesen bequem z.B. daheim durchführen. Durch die Übertragung der während des Tests gewonnen Daten an die zentrale Datenbank hat insbesondere der den Patienten behandelnde Arzt die Möglichkeit, die während des Tests gewonnen Daten einzusehen und dementsprechend die Behandlung des Patienten zu modifizieren.

Weitere vorteilhafte Ausgestaltungen des medizinischen Systems ergeben sich aus den Unteransprüchen.

Die Aufgabe wird auch gelöst durch ein tragbares Gerät zur Bestimmung psychomotorischer Fähigkeiten, aufweisend
- eine Schreibfläche, auf der handschriftlich Buchstaben oder Zahlen geschrieben werden,
- Mittel zum Erkennen der handschriftlich geschriebenen Buchstaben oder Zahlen,
- ein Sichtfeld zum Wiedergeben der erkannten handschriftlichen Buchstaben oder Zahlen als Druckbuchstaben und
- Mittel zum Anschluss an ein Informationsübertragungsnetz.

Der Patient schreibt mit beispielsweise einem Stift handschriftlich Zahlen oder vorzugsweise Buchstaben auf die Schreibfläche des tragbaren Gerätes. Bei leserlicher Handschrift werden die Zahlen bzw. Buchstaben von den Mitteln zum Erkennen der handschriftlich geschriebenen Buchstaben oder Zahlen erkannt und auf der Sichtfläche als Druckbuchstaben wiedergegeben. Der Patient oder ein den Patienten betreuende Person notiert die Anzahl der geschriebenen Buchstaben bzw. Zahlen und die Anzahl der nicht erkannten Buchstaben bzw. Zahlen und berechnet daraus eine Fehlerquote. Insbesondere die Fehlerquote kann mit dem tragbaren Gerät, wenn es an das Internet anschließbar ist, beispielsweise einer zentralen Datenbank übermittelt werden.

Nach einer vorteilhaften Ausführungsform ist das tragbare Gerät derart ausgeführt, dass es die Fehlerquote automatisch berechnet.

Ein Ausführungsbeispiel der Erfindung ist in den beigelegten schematischen Zeichnungen dargestellt. Es zeigen:
- Fig. 1: ein das erfindungsgemäße medizinische System umfassende Schaubild und
- Fig. 2: einen Personal Digital Assistant (PDA).

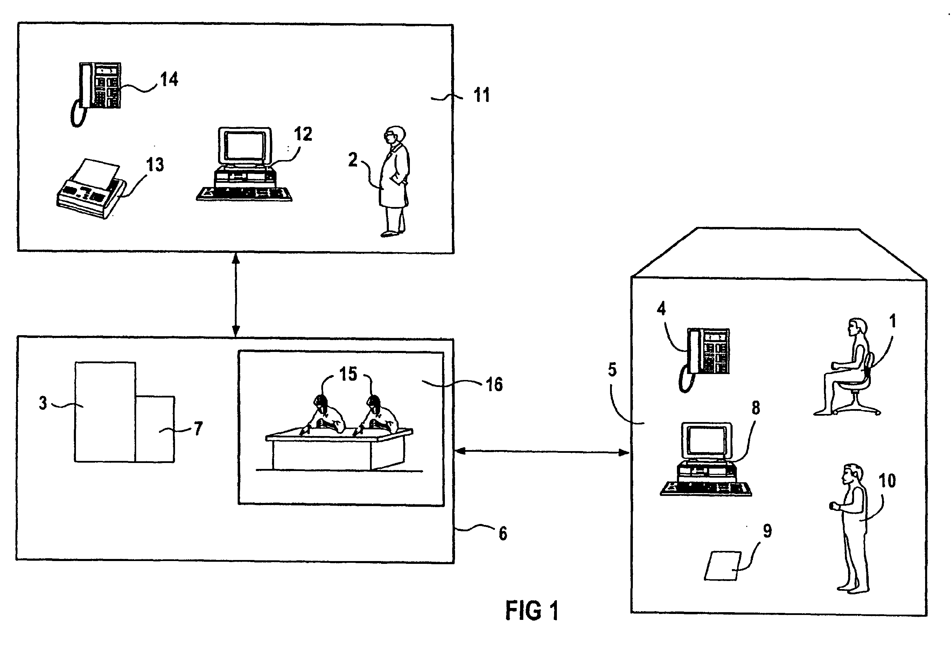

Ein in der Fig. 1 schematisch dargestellter Patient 1 nimmt regelmäßig ein an seinem zentralen Nervensystem angreifendes, in der Fig. 1 nicht dargestelltes Medikament ein. Damit ein den Patienten 1 behandelnder Arzt 2 eine Unter- oder Überdosierung des Medikamentes oder Nebenwirkungen des Medikamentes so schnell als möglich erkennen kann, führt der Patient 1 regelmäßig, im Falle des vorliegenden Ausführungsbeispieles drei mal wöchentlich, in einer außerklinischen Umgebung einen psychomotorischen Test durch, bei dem im Falle des vorliegenden Ausführungsbeispieles die Reaktionszeit, das Gedächtnis, die Sprachverständlichkeit und die Lesbarkeit der Handschrift des Patienten 1 getestet wird. Insbesondere bei Nebenwirkungen oder einer Überdosierung des Medikaments ist zu erwarten, dass sich u.a. die Reaktionszeit, das Gedächtnis, die Sprachverständlichkeit und/oder die Lesbarkeit der Handschrift des Patienten 1 verschlechtern.

Damit der psychomotorische Test in für den Patienten 1 praktischer Weise durchgeführt werden kann, kontaktiert der Patient 1, wenn er den psychomotorischen Test durchführen will, eine im Falle des vorliegenden Ausführungsbeispiels mit zur Spracheingabe und Sprachwiedergabe geeigneten Mitteln versehene Datenbank 3 mit einem Telefon 4. Im Falle des vorliegenden Ausführungsbeispieles befindet sich der Patient 1 während des psychomotorischen Tests in seinem Haus 5. Selbstverständlich kann der Patient 1 diesen Test beispielsweise auch am Arbeitsplatz, im Freien, wie z.B. mit einem in der Fig. 1 nicht dargestellten mobilen Telefon, oder auch in einer Telefonzelle durchführen, so lange er Zugang zu einem Telefon hat.

Die Datenbank 3 wird im Falle des vorliegenden Ausführungsbeispiels von einem Service-Anbieter 6 betrieben, der die Datenbank 3 pflegt. Des Weiteren ist der Datenbank 3 eine Auswerteeinrichtung 7 zugeordnet, die die aufgrund des psychomotorischen Tests gewonnen Daten auswertet.

Bei der Kontaktierung der Datenbank 3 wird zuerst die Identität des Patienten 1 mittels einer Identifikationsnummernkombination, die der Patient 1 mit dem Telefon 4 durchgibt und die Datenbank 3 mit einer geeigneten Programmprozedur auswertet, bestimmt. Anschließend wird im Falle des vorliegenden Ausführungsbeispieles ein Reaktionstest durchgeführt, indem die Datenbank 3 ein in der Datenbank 3 gespeichertes Testprogramm startet und dem Patienten 1 mittels des Telefons 4 eine Zahl zwischen 0 und 9 nennt, die der Patient 1 mit den Wähltasten des Telefons 4 bestätigt. Dieser Test wird mit verschiedenen Zahlen neunmal wiederholt. Für den Reaktionstest misst die Auswerteeinrichtung 7 die jeweilige Reaktionszeit, die der Patient 1 benötigt, um nach Nennung der Zahl die entsprechende Wähltaste des Telefons 3 zu drücken und vergleicht diese Reaktionszeit mit einer durchschnittlichen in der Datenbank 3 gespeicherten Reaktionszeit, die der Patient 1 für denselben Test vor der Behandlung mit dem Medikament benötigte. Sollten sich die Reaktionszeiten im Falle des vorliegenden Ausführung bei mehr als drei Messungen um mehr als eine Sekunde verschlechtert haben, wird das Ergebnis des Reaktionstests von der Auswerteeinrichtung 7 als bedenklich und ansonsten als unbedenklich eingestuft. Außerdem werden die einzelnen Reaktionszeiten, die Veränderungen der Reaktionszeiten im Vergleich zu der durchschnittlichen, vor der Behandlung ermittelten Reaktionszeit und das Ergebnis des Reaktionstests in der Datenbank 3 gespeichert.

Anschließend wird im Falle des vorliegenden Ausführungsbeispieles ein Test zur Bestimmung der Sprachverständlichkeit des Patienten 1 durchgeführt, indem die Datenbank 3 ein weiteres in der Datenbank 3 gespeichertes Testprogramm startet und dem Patienten 1 einen auf der Datenbank 3 gespeicherten Satz vorspricht, den der Patient 1 wiederholt. Der von dem Patienten 1 wiederholte Satz wird von der Datenbank 3 aufgenommen und von der Auswerteeinrichtung 7 mittels einer Sprachmusteranalyse auf Vollständigkeit und Verständlichkeit überprüft und mit einer Sprachmusteranalyse des Patienten 1 verglichen, welche vor der Behandlung mit dem Medikament durchgeführt wurde. Bei einer deutlichen Verschlechterung der Sprachverständlichkeit oder einer Unvollständigkeit des Satzes wird der Test zur Sprachverständlichkeit als bedenklich und ansonsten als unbedenklich eingestuft. Das Ergebnis dieses Tests wird ebenfalls in der Datenbank 3 gespeichert.

Danach wird im Falle des vorliegenden Ausführungsbeispieles ein Gedächtnistest durchgeführt, indem die Datenbank 3 ein weiteres in der Datenbank 3 gespeichertes Testprogramm startet und dem Patienten 1 eine zehnstellige Zahl nennt, die dieser mittels der Wähltasten wiedergibt. Die Auswerteeinrichtung 7 vergleicht dabei die von der Datenbank 3 vorgegebene mit der von dem Patienten 1 eingegebenen zehnstelligen Zahl und ermittelt die Anzahl der falsch eingegebenen Ziffern. Anschließend vergleicht die Auswerteeinrichtung 7 die Anzahl der falsch eingegebenen Ziffern mit einer durchschnittlichen Anzahl von falsch eingegebenen Ziffern für eine Reihen derselben Tests, die der Patient 1 vor der Behandlung mit dem Medikament durchführte. Sollte sich die Anzahl der falsch eingegebenen Ziffern um mehr als zwei erhöht haben, wird im Falle des vorliegenden Ausführungsbeispiels der Gedächtnistest von der Auswerteeinrichtung 7 als bedenklich und ansonsten als unbedenklich eingestuft. Die Anzahl der falsch eingegebenen Ziffern und das Ergebnis des Gedächtnistests werden in der Datenbank 3 gespeichert.

Eine andere Möglichkeit der außerklinischen Durchführung eines psychomotorischen Tests besteht in der Verwendung eines an das Internet angeschlossenen Rechners 8. Im Falle des vorliegenden Ausführungsbeispiel kontaktiert der Patient 1 mit dem in seinem Haus 5 angeordneten und an das Internet angeschlossen Rechner 8 eine ihm zugeordnete und vom Service-Anbieter 6 gepflegte WWW-Homepage und führt einen Gedächtnistest durch. Im Falle des vorliegenden Ausführungsbeispiels hat der Patient 1 nach der Kontaktierung der WWW-Homepage eine Minute Zeit, einen auf der WWW-Homepage wiedergegebenen Satz zu lesen. Nach einer Minute wird dieser Satz ausgeblendet und der Patient 1 muss mittels einer dem Rechner 8 zugeordneten Tastatur den Satz in den Rechner 8 eingeben, der auch in der Datenbank 3 gespeichert wird. Anschließend vergleicht die Auswerteeinrichtung 7 den von dem Patienten 1 eingegeben Satz mit dem ursprünglichen Satz und ermittelt die Fehlerquote, die wiederum mit einer Fehlerquote des Patienten 1 verglichen wird, die vor der Behandlung mit dem Medikament ermittelt wurde. Steigt im Falle des vorliegenden Ausführungsbeispiels die Fehlerquote um mehr als 10%, wird der Gedächtnistest als bedenklich und ansonsten als unbedenklich eingestuft.

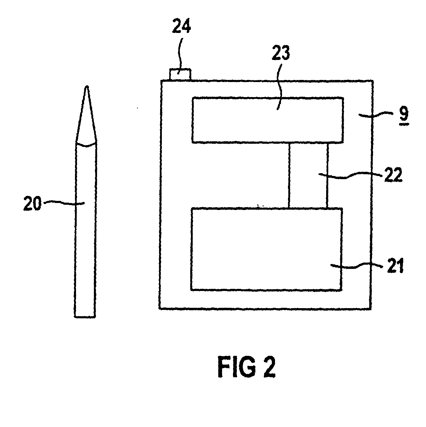

Eine dritte Form der außerklinischen Durchführung eines psychomotorischen Tests stellt ein Test zur Ermittlung der Lesbarkeit der Handschrift des Patienten 1 dar, der mittels eines ebenfalls in der Fig. 2 schematisch dargestellten Personal Digital Assistant (PDA) 9 durchgeführt wird. Im Falle des vorliegenden Ausführungsbeispiels wird der PDA 9 dem Patienten 1 von einer den Patienten 1 betreuende Person 10 zur Verfügung gestellt. Mittels eines in der Fig. 2 schematisch dargestellten Stifts 20 schreibt der Patient 1 auf einer Schreibfläche 21 des PDAs 9 handschriftlich verschiedene von der Person 10 genannte Buchstaben, die mit geeigneten Mitteln 22 zum Erkennen von handschriftlich geschriebenen Buchstaben des PDAs 9 bei einer nur unwesentlich gestörten psychomotorischen Fähigkeit des Patienten 1 erkannt und als Druckbuchstaben auf dem Sichtfeld 23 des PDAs 9 wiedergegeben werden. Die Person 10 notiert während dieses Tests die Anzahl der unleserlich geschriebenen Buchstaben, die Anzahl der genannten Buchstaben und die Zeitdauer, die der Patient 1 für das Schreiben der Buchstaben benötigt. Nach Beendigung des Tests zur Ermittlung der Lesbarkeit der Handschrift des Patienten 1 übermittelt die Person 10 im Falle des vorliegenden Ausführungsbeispiels die Ergebnisse des Schreibtests an die Datenbank 3 über das Internet. Dazu ist der PDA 9 mit einer geeigneten Schnittstelle 24 zum Anschluss an das Internet versehen. Anschließend berechnet die Auswerteeinrichtung 7 aus diesen Angaben eine Fehlerquote, die die Auswerteeinrichtung 7 mit einer vor der Behandlung mit dem Medikament ermittelten Fehlerquote des Patienten 1 vergleicht. Außerdem vergleicht die Auswerteeinrichtung 7 die Zeitdauer, die der Patient 1 zum Schreiben der Buchstaben benötigte, mit einer Zeitdauer, die der Patient 1 für das Schreiben der Buchstaben ohne medikamentöser Behandlung benötigte. Im Falle des vorliegenden Ausführungsbeispiels stuft die Auswerteeinrichtung 7 das Ergebnis des Tests zur Lesbarkeit der Handschrift des Patienten 1 als bedenklich ein, wenn die Fehlerquote und/oder die Zeitdauer zum Schreiben der Buchstaben um mehr als 10% steigen. Ansonsten stuft die Auswerteeinrichtung 7 den Schreibtest als unbedenklich ein.

Alternativ zu dem manuellen Aufschreiben der Anzahl der unleserlichen Buchstaben, der Anzahl der genannten Buchstaben und der zum Schreiben der Buchstaben benötigten Zeitdauer, kann der PDA 9 auch derart ausgeführt sein, dass er diese Informationen automatisch erfasst oder die Fehlerquote automatisch berechnet.

Sollte einer der obenstehend beschriebenen Tests von der Auswerteeinrichtung 7 als bedenklich eingestuft worden sein, verschickt die Datenbank 3 automatisch eine e-mail an einen im Falle des vorliegenden Ausführungsbeispiels in einer Arztpraxis 11 des Arztes 2 angeordneten Rechner 12, um dem Arzt 2 das bedenkliche Ergebnis mitzuteilen. Insbesondere bei einer gestörten Internetverbindung kann der Arzt 2 auch mit einem in der Arztpraxis 11 angeordneten Faxgerät 13 oder Telefon 14 von einem der Personen 15, die in einem dem Service-Anbieter 6 zugeordneten Call-Center 16 arbeiten, von dem bedenklichen psychomotorischen Test informiert werden. Die Personen 15 können u.a. auch bei Fragen bezüglich des psychomotorischen Tests von dem Arzt 2, dem Patienten 1 oder der Person 10 kontaktiert werden.

Außerdem ist im Falle des vorliegenden Ausführungsbeispiels vorgesehen, dass der Arzt 2 die Datenbank 3 über das Internet kontaktieren kann, um beispielsweise den psychomotorischen Test insbesondere aufgrund eines bedenklichen Ergebnisses zu überprüfen und wenn nötig den Patienten 1 zu kontaktieren und/oder schnell und zuverlässig Hilfsmaßnahmen für den Patienten 1 einzuleiten.

Des Weiteren ist im Falle des vorliegenden Ausführungsbeispiels vorgesehen, dass die Datenbank 3 automatisch den Arzt 2 per e-mail, telefonisch oder per Fax informiert, wenn nach einem psychomotorischen Test mehr als zwei Tage vergangen sind, ohne dass der Patient 1 einen neuen psychomotorischen Test durchgeführt hat. Folglich kann in solch einem Fall der Arzt 2 den Patienten 1 an die Wichtigkeit des psychomotorischen Tests erinnern, um eine gute Behandlung des Patienten 1 mit dem Medikament sicher zu stellen.

Der Service-Anbieter 6 muss im Übrigen nicht notwendigerweise ein Call-Center 16 betreiben. Die Datenbank 3 muss auch nicht notwendigerweise von einem Service-Anbieter 6 betrieben werden, sie kann beispielsweise auch in der Arztpraxis 11 angeordnet sein und von dem Arzt 2 oder einem Kundendienst betreut werden.

Es ist auch möglich, dass einer der Personen 15 des Call-Centers 16 oder die Datenbank 3 den Patienten 1 kontaktiert, damit dieser den psychomotorischen Test durchführt.

Die Auswerteeinrichtung 7 kann auch ein Expertensystem umfassen, welches die aufgrund des psychomotorischen Tests übermittelten Daten anhand von Regelsystemen oder auf Wahrscheinlichkeiten basierend interpretiert. Dabei ist eine Individualisierung des Expertensystems auf den Patienten vorsehbar, d.h. das Expertensystem lernt den Patienten, den es überwacht, im Überwachungsprozess immer besser kennen, es trifft dazu als lernendes System ständig Prognosen über zu erwartende zukünftige Daten, die es mit den wahren Daten vergleicht. Damit wird eine individualisierte Überwachung erreicht.

Der psychomotorische Test muss auch nicht notwendigerweise regelmäßig durchgeführt werden.

Die Beschreibung des psychomotorischen Tests ist exemplarisch zu verstehen. Insbesondere müssen nicht alle der genannten Geräte Telefon 4, Rechner 8 oder PDA 9 für den psychomotorischen Test verwendet werden. Es können auch weitere, in den Fig. 1 und 2 nicht gezeigte Geräte oder Methoden zur Durchführung des psychomotorischen Tests verwendet werden. Auch eine Betreuung des Patienten 1 durch die Person 10 ist für die Erfindung nicht notwendig. Die in der Fig. 2 gezeigte Ausführung eines PDAs 9 ist auch nur exemplarisch zu verstehen. Ein PDA kann insbesondere auch ein Laptop sein.

Für das erfinderische Verfahren ist eine automatische Auswertung des psychomotorischen Tests mittels der Auswerteeinrichtung 7 oder ein automatisches Informieren des Arztes 2 bei einem bedenklichen Ergebnis des psychomotorischen Tests optional. Es können auch andere Personen informiert werden. Auch ein Informieren des Arztes darüber, dass der Patient 1 einen psychomotorischen Test nicht ausführt ist sowohl für das Verfahren als auch für das medizinische System optional. Ferner können auch weitere Personen informiert werden.

## Patentansprüche

1. Verfahren zur Bestimmung psychomotorischer Fähigkeiten eines Patienten (1) in außerklinischer Umgebung, aufweisend folgende Verfahrensschritte:
- Kontaktieren einer Datenbank (3) mit einem Telefon (4), mit einem an ein Informationsübertragungsnetz anschließbaren Rechner (8) oder mit einem Personal Digital Assistant (PDA),
- Durchführen eines psychomotorischen Tests mit dem Telefon (4), dem Rechner (8) oder dem Personal Digital Assistant (PDA), und
- Übermitteln der während des psychomotorischen Tests gewonnen Daten an eine Datenbank (3), welche an einem Ort (6) angeordnet ist, der von dem Ort (5), an dem der psychomotorische Test durchgeführt wird, verschieden ist.

2. Verfahren zur Bestimmung psychomotorischer Fähigkeiten nach Anspruch 1, bei welchem die Datenbank (3) von einem Service-Anbieter (6) betrieben wird.

3. Verfahren nach Anspruch 1 oder 2, bei welchem der psychomotorische Test einen Reaktionstest, einen Gedächtnistest, einen Test zur Bestimmung der Lesbarkeit der Handschrift des Patienten (1) und/oder einen Test zur Bestimmung der Sprachverständlichkeit des Patienten (1) umfasst.

4. Verfahren zur Bestimmung psychomotorischer Fähigkeiten nach einem der Ansprüche 1 bis 3, bei welchem die Daten insbesondere automatisch mit einer der Datenbank (3) zugeordneten Auswerteeinrichtung (7) ausgewertet werden.

5. Verfahren zur Bestimmung psychomotorischer Fähigkeiten nach Anspruch 4, bei welchem die Auswerteeinrichtung (7) Mittel zur Sprachmusteranalyse, Mittel zur Zeitmessung, Mittel zum Vergleichen von Buchstabenfolgen, Mittel zum Vergleichen von Zahlenfolgen und/oder ein Expertensystem umfasst.

6. Verfahren zur Bestimmung psychomotorischer Fähigkeiten nach einem der Ansprüche 1 bis 5, bei welchem die Datenbank (3) über ein Informationsübertragungsnetz, insbesondere über das Internet kontaktiert werden kann.

7. Verfahren zur Bestimmung psychomotorischer Fähigkeiten nach einem der Ansprüche 4 bis 6, bei welchem die Datenbank (3) automatisch den Patienten (1) und/oder wenigstens eine weitere Person (2) verständigt, wenn die ausgewerteten Daten bedenklich sind.

8. Verfahren zur Bestimmung psychomotorischer Fähigkeiten nach einem der Ansprüche 1 bis 7, bei welchem die Datenbank (3) den Patienten (1) und/oder wenigstens eine weitere Person (2) automatisch verständigt, wenn nach einem psychomotorischen Test eine vorbestimmte Zeitdauer verstrichen ist, ohne dass ein weiterer psychomotorischer Test durchgeführt wurde.

9. Vorrichtung zur Bestimmung psychomotorischer Fähigkeiten eines Patienten (1) in außerklinischer Umgebung (5), aufweisend eine
- Datenbank (3), welche an einem Ort (6) angeordnet ist, der von dem Ort (5), an dem ein psychomotorischer Test zur Bestimmung der psychomotorischen Fähigkeiten durchgeführt wird, verschieden ist, und welche während des psychomotorischen Tests ermittelte Daten des Patienten (1) speichert,
- eine der Datenbank (3) zugeordnete Auswerteeinrichtung (7) zur Auswertung dieser Daten und
- eine Alarmeinrichtung (12 bis 14) zur Erzeugung eines Alarmsignal, wenn die ausgewerteten Daten bedenklich sind.

10. Vorrichtung zur Bestimmung psychomotorischer Fähigkeiten eines Patienten (1), aufweisend
- eine Datenbank (3), die
- an ein Informationsübertragungsnetz anschließbar ist,
- mit einem Telefon (4) oder einem Rechner (8) über das Informationsübertragungsnetz kontaktierbar ist und
- mit dem Rechner (8) derart kommunizieren kann, dass ein von der zentralen Datenbank (3) gesteuerter psychomotorischer Test durchführbar ist und
- eine der zentralen Datenbank (3) zugeordnete Auswerteeinrichtung (7) zur Auswertung des durchgeführten psychomotorischen Tests.

11. Vorrichtung nach Anspruch 9 oder 10, bei der die Auswerteeinrichtung (7) Mittel zur Sprachmusteranalyse, Mittel zur Zeitmessung, Mittel zum Vergleichen von Buchstabenfolgen, Mittel zum Vergleichen von Zahlenfolgen und/oder ein Expertensystem umfasst.

12. Vorrichtung nach einem der Ansprüche 9 bis 11, bei der die zentrale Datenbank (3) derart ausgeführt ist, dass sie bei einem bedenklichen ausgewerteten psychomotorischen Test automatisch wenigstens eine Person (1, 2) verständigt.

13. Vorrichtung nach einem der Ansprüche 9 bis 12, bei der die zentrale Datenbank (3) derart ausgeführt ist, dass sie automatisch wenigstens eine Person (1, 2) verständigt, wenn nach einer Durchführung eines psychomotorischen Tests eine vorbestimmte Zeitdauer verstrichen ist, ohne dass ein weiterer psychomotorischer Test durchgeführt wird.

14. Vorrichtung nach einem der Ansprüche 9 bis 13, bei der die Datenbank (3) über das Internet kontaktierbar ist.

15. Tragbares Gerät zur Bestimmung psychomotorischer Fähigkeiten, aufweisend
- eine Schreibfläche (21), auf der handschriftlich Buchstaben oder Zahlen geschrieben werden,
- Mittel (22) zum Erkennen der handschriftlich geschriebenen Buchstaben oder Zahlen,
- ein Sichtfeld (23) zum Wiedergeben der erkannten handschriftlichen Buchstaben oder Zahlen als Druckbuchstaben und
- Mittel (24) zum Anschluss an ein Informationsübertragungsnetz.

16. Tragbares Gerät nach Anspruch 15, das derart ausgeführt ist, dass es automatisch eine Fehlerquote aus der Anzahl der handschriftlichen Buchstaben bzw. Zahlen und den nicht erkannten Buchstaben bzw. Zahlen berechnet.

17. Tragbares Gerät nach Anspruch 16, bei dem die Fehlerquote nach Anschluss an das Informationsübertragungsnetz an eine zentrale Datenbank (3) übertragen und in der zentralen Datenbank (3) gespeichert wird.

18. Verfahren zur Bestimmung psychomotorischer Fähigkeiten eines Patienten (1), aufweisend folgende Verfahrensschritte:
- Kontaktieren einer zentralen Datenbank (3) mit einem Telefon (4) oder mit einem Rechner (8) über ein Informationsübertragungsnetz,
- Durchführen eines von der zentralen Datenbank (3) gesteuerten psychomotorischen Tests mit dem Telefon (4) oder dem Rechner (8), und
- Auswerten von während des psychomotorischen Tests ermittelten Daten von einer der zentralen Datenbank (3) zugeordneten Auswerteeinrichtung (7).
